# EUROPÄISCHE PATENTANMELDUNG

(11) **EP 0 598 935 A1**
(43) Veröffentlichungstag der Anmeldung: **01.06.1994**
(21) Anmeldenummer: 92119963.4
(22) Anmeldetag: 24.11.1992
(51) Int. Cl.: C12N 15/86, C12N 15/11, A61K 31/70, C12N 5/10

(54) **Expressionsvektoren und deren Verwendung zur Darstellung HIV-resistenter menschlicher Zellen für therapeutische Anwendungen**

(71) Anmelder: BAYER AG, 51368 Leverkusen (DE)
(72) Erfinder: Kretschmer, Axel, Dr., W-5060 Bergisch Gladbach (DE); Antonicek, Horst-Peter, Dr., W-5060 Bergisch Gladbach (DE); Baumgarten, Jörg, Dr., W-5600 Wuppertal (DE); Loebberding, Antonius, Dr., W-5600 Wuppertal (DE); Mielke, Burkhard, Dr., W-5090 Leverkusen (DE); Springer, Wolfgang, Dr., W-5600 Wuppertal (DE); Stropp, Udo, Dr., W-5657 Haan (DE); Struck, Mark-Michael, Dr., CH-3013 Bern (CH); Biesert, Lothar, Dr., W-6000 Frankfurt 50 (DE); Rübsamen-Waigmann, Helga, Prof. Dr., W-6232 Bad Soden (DE); Suhartono, Hary, Dr., W-6000 Frankfurt 50 (DE); Hausner, Thomas-Peter, Dr., W-5000 Köln 80 (DE)

(57) **Zusammenfassung**

Die vorliegende Erfindung betrifft die gezielte Blockierung der genetischen Information der viralen Messenger-Ribonukleinsäure (mRNA) für die Virusreplikation (z.B. von HIV) in transfizierten, hämatopoetischen Zellen mit besonders geeigneten Expressionssystemen, die eine komplementäre "Antisense-RNA" exprimieren.

## Beschreibung

Die vorliegende Erfindung betrifft die gezielte Blockierung der genetischen Information der viralen Messenger-Ribonukleinsäure (mRNA) für die Virusreplikation (z.B. von HIV) in transfizierten, hämatopoetischen Zellen mit besonders geeigneten Expressionssystemen, die eine komplementäre "Antisense-RNA" exprimieren.

Insbesondere betrifft die Erfindung Expressionsvektoren kodierend für Antisense-RNA Sequenzen, die im Gegensatz zum Stand der Technik eine vollständige Hemmung der HIV-1-Replikation und eine deutliche Hemmung der HIV-2 Replikation in gentechnisch veränderten menschlichen Zellen darstellen. Die hier beschriebenen genetischen Einheiten der Expressionsvektoren sind in besonderer Weise für die somatische Gentherapie von HIV/AIDS-Erkrankungen geeignet, weil sie hämatopeotische Zellen vor HIV-induzierten zytopathischen Effekten schützen. Dadurch kann die Immuninsuffizienz in HIV infizierten Menschen vermieden werden.

Die Virusvermehrung in einer infizierten Zelle wird durch die Gene des Virusgenoms gesteuert. Dabei werden regulatorische Genprodukte und Strukturproteine für den Aufbau infektiöser Viruspartikel zur Virusvermehrung von diesen Virusgenen durch Transkription und Translation gebildet. Das essentielle Intermediat für diesen Prozeß ist die Messenger-Ribonukleinsäure (mRNA), die die Information für die Bildung der regulatorischen und Strukturproteine vermittelt.

Eine Blockierung der Messenger-Ribonukleinsäure in einer hochspezifischen Weise, die allein auf der Einzigartigkeit der molekularen Struktur der viralen mRNA, nämlich der virusspezifischen Nukleobasensequenz dieses Moleküles, beruht, kann mit einer Ribonukleinsäure komplementärer Basensequenz erfolgen: Durch sequenzspezifische Nukleobasenpaarung, wie sie analog für die Desoxyribonukleinsäure beschrieben wurde, wird die vormals einzelsträngige mRNA in einer doppelsträngigen RNA komplexiert, und die genetische Information der Nukleobasensequenz für die Proteinbiosynthese unzugänglich. Der zweite Schritt der Genexpression, die Translation, wird dadurch gehemmt. Die komplementäre Ribonukleinsäure wird als Antisense-RNA bezeichnet.

Der Mechanismus der Hemmung der Genexpression durch Wechselwirkungen der Antisense-RNA mit der Ziel-mRNA ist vielschichtig und weitgehend ungeklärt. Eine Erklärung ist die Hemmung des RNA-Splicing und RNA-Processing im Zellkern, detaillierte Untersuchungen darüber sind jedoch nicht bekannt. Publiziert wurde der Adenosin → Inosin-Austausch in doppelsträngiger RNA und als Folge des Austausches die Translationshemmung diskutiert. Der gehinderte RNA-Transport durch die perinukleare Membran an den Ort der Proteinbiosynthese und die Maskierung der Ribosomen/RNA-Wechselwirkung durch doppelsträngige RNA mögen weitere Beiträge zur Hemmung der Genexpression durch Antisense-RNA leisten.

Die Hemmung von Stoffwechselprozessen durch Antisense-RNA in natürlich vorkommenden biologischen Systemen wie Bakterien und einigen eukaryotischen, vielzelligen Organismen [K.M.Takayama und M. Inouye, Critical Reviews in Biochemistry and Molecular Biology 25, 155 (1990)] sowie in künstlich durch Gentransfer erzeugten, transgenen Organismen [J. Mol et al., FEBS Letters 268, 427 (1990)] ist beschrieben worden.

So ist auch über Versuche berichtet worden, in Pflanzen [M. Cuozzo et al., Biotechnology 6, 549 (1988)] und tierischen Zellen [T. Rüden und E. Gilboa, J. Virol. 63, 677 (1989), EP 252 940] nach Einführung eines Antisense-RNA exprimierenden Gens, virusresistente Organismen darzustellen. Es gibt aber keine überzeugenden, wissenschaftlich nachgewiesenen Zusammenhänge von Antisense-RNA-Expression und Virusresistenz, wie die Autoren in diesen Publikationen selbst ausführen. Insbesondere ist bei mikroinjizierten Antisense-RNA exprimierenden Plasmiden bei transienter Expression die antivirale Wirkung schwach oder führt zu nicht schlüssig auswertbaren, antiviralen Wirkungen [K. Rittner et al., Nucleic Acids Research 19, 1421 (1991)]. Auch ist von Antisense-RNA exprimierenden Retrovirus-Vektoren [R.Y-L. To et al. in: Gene Regulation: Biology of Antisense RNA and DNA ed. R.P. Erickson and J.G. Izant, Raven Press Ltd. New York 1992] zur Erzeugung virusresistenter Zellen berichtet worden. Diese retroviralen Vektoren bergen das Risiko, malignes Tumorwachstum auszulösen, weil sie oncogenähnliche Gensequenzen enthalten, und die retrovirusabhängige Oncogenese mannigfaltig belegt ist.

Im Gegensatz zu den publizierten Arbeiten über Antisense-RNA zur Kontrolle der Virusreplikation werden in der hier vorliegenden Erfindung Expressionskonstrukte beschrieben, die sich durch die besondere Art ihrer Promotorkonstrukte in Kombination mit den besonders ausgewählten Bereichen der viralen DNA in Antisense-Orientierung auszeichnen und dadurch antivirale Wirkungen mit einer Wirkqualität und Wirkquantität ergeben, die überraschend sind und den bisher bekannten Umfang bei weitem übertreffen. Die Kombination von Expressionsvektorbestandteilen; 5'-flankierende DNA-Sequenz, Promotor, Insert/Antisense-DNA und 3'-Prozessiersignalsequenzen führten zu der erstmals bei retroviralen Infektionen sicher nachweisbaren und deutlich ausgeprägten Virusresistenz transzifierter, Antisense-RNA exprimierender Zellen. Der Stand der Technik mit anderen Vektorsystemen zur endogenen Antisense-RNA Expression und Retrovirusresistens ergibt dagegen schwache, nur vorübergehend auftretende antivirale Effekte [z.B. T. Rüden und E. Gilboa, J. Virol, 63 677 (1989), EP 252 940, G. Sczakiel et al., Biochem.

Biophys. Res. Com. 169, 643 (1990) O. I. Mirosknichenko et al., Gene 84, 83 (1989)] [T. Shimada et al. Antiviral Chemistry and Chemotherapy 2, 133 (1991); G. Sczakiel et al. J. Virol. 66, 5576 (1992)], oder ist mit den oben genannten Risiken behaftet (retroviraler Gen-transfer) oder ist unpraktikabel, weil Mikroinjektionen erforderlich sind.

Die Erfindung betrifft Expressionsvektorkonstrukte zur Expression von Antisense-RNA, welche Hybridpromotorgensequenzen enthalten und eine starke konstitutive oder bei Befall durch Pathogene induzierbare Promotoraktivität aufweisen.

Dies sind zum Beispiel solche Expressionsvektorkonstrukte die subgenomische Gensequenzen eines pathogenen Virus in 3' → 5' Orientierung (Antisense Orientierung des (+)-DNA Stranges) unter der Kontrolle des Promotors zur Expression von Antisense-RNA enthalten.

Gut geeignet im Sinne der vorliegenden Erfindung sind solche die provirale, subgenomische DNA in Antisense-Orientierung von Retroviren wie HIV-1 und HIV-2 oder von Oncoviren wie HTLV-I, HTLV-II oder von Oncogenen wie abl, erb A, erb B, fms, fos, myb, myc, ras, sis, src enthalten.

Bevorzugt sind Expressionsvektorkonstrukte, die HIV-1 provirale DNA in Antisense-Orientierung der Nukleotide 7514→474 oder 5786→474 oder 2096→474 oder 3825→681oder 5786→4158 oder 5746→4648 oder 2369→1635 oder 3226→3003 oder 2099→678 oder 3829→2099 oder 4647→4157 oder 4157→3820 enthalten.

Es sind auch Expressionsvektorkonstrukte einsetzbar, die subgenische provirale Gensequenzen oder Oncogensequenzen in 3'→ 5' Orientierung für die Expression kürzerer Antisense-RNA Transkripte enthalten.

Hier sind solche Expressionsvektorkonstrukte zu nennen, die provirale Gensequenzen von HIV-1 der Nukleotide 605→455 oder 670→440 oder 825→535 oder 6070→5800 oder 5640→5020 oder 5600→5040 oder 8690→8300 oder 9160→8800 enthalten.

Bevorzugt sind Expressionvektrorokonstrukte, die zwei, drei oder mehr subgenomische Gensequenzen von einem Virus oder von mehreren Viren enthalten.

Die Erfindung betrifft auch transfizierte Zellen, die oben genannten Expressionsvektorkonstrukte enthalten.

Ebenfalls zu der Erfindung gehört die Verwendung der genannten Expressionsvektorkonstrukten zur Herstellung von Arzneimitteln wie auch Arzneimittel enthaltend einen oder mehrere der Expressionsvektorkonstrukte.

Mit den hier beschriebenen Expressionsvektor-Konstrukten wurde in relevanten, menschlichen Wirtszellen für die HIV-Replikation eine antivirale Wirkung gezeigt, die einen vollständigen Schutz vor HIV-1 Replikation in menschlichen Zellen über 60 Tage hinaus darstellt und die außerdem signifikant die Hemmung der HIV-2 Replikation bewirkt.

Mit der vorliegenden Erfindung wird der Aufbau und die Zusammensetzung von Expressionsvektorkonstrukten beschrieben, die Messenger-Ribonukleinsäuretranskripte in transfizierten Zellen exprimieren, die komplementär zu mRNA Sequenzen unerwünschter viraler Gene, hier am Beispiel der Gene GAG, POL, VIF, REV, TAT und VPU [W. A. Haseltine et al., Scientific American USA, 1988, 34 bis 42] des Humanen Immunschwächevirus HIV-1 sind. Außerdem ist die beschriebene Antisense-RNA Sequenz ausreichend homolog gegen HIV-2 (z.B. Isolat HIV-2 D194, in: Human Retroviruses and AIDS, eds. Myers et al. Los Alamos National Lab., 1992, and EMBL accession no. X52223) im Bereich der Gene GAG, POL und VIF, um auch gegen HIV-2 Isolate antivirale Wirkungen zu erzielen. Die Expressionsvektorkonstrukte zeichnen sich durch die besondere Art der Anordnung von ausgewählten Promotorsequenzen und Hybridpromotorsequenzen (z.B. CMV-IE/Metallothionein I) mit subgenomischen HIV-1 DNA Fragmenten in Antisenseorientierung (3' → 5') aus, die die Transkription mehrerer Antisense-RNA Transkripte durch RNA splicing in konstitutiver oder induzierbarer Weise ergeben. So gebildete Antisense-RNA Transkripte zeigten überraschenderweise eine drastische oder vollständige Hemmung der HIV-1 Replikation in menschlichen Zellen (HUT 78, U 937 zum Beispiel). Auch wurde eine deutliche Hemmung der HIV-2 Replikation festgestellt. HIV-2 weist in den hier beschriebenen Expressionsvektorkonstrukten eine ausreichende RNA-Sequenzhomologie auf, so daß die Hemmung der Virusreplikation durch Antisense-RNA zum tragen kommt. Die Erfindung umfaßt somit eine Methode zur Hemmung der Replikation von Retroviren am Beispiel HIV-1 und HIV-2, die geeignet ist, hämatopoetische Zellen und damit wichtige Zellen der menschlichen, zellvermittelten Immunreaktion (z.B. T-Zellen, Monozyten) vor zellschädigender und zellabbauender Virusvermehrung durch eine bestimmte Weise der Expression antiviral wirksamer Antisense-RNA zu schützen.

Zellen des Immunsystems vor der zellschädigenden Wirkung der Retrovirusvermehrung, wie sie von der HIV-1 und HIV-2 Infektion bekannt ist, durch die Expression antiviraler Antisense-RNA resistent zu machen, wurde wie folgt experimentell umgesetzt.

### Promotoren:

Expressionsvektoren wurden konstruiert, die Promotoren enthalten, die in hämatopoetischen Zellen konstitutiv und stark exprimieren sollten wie z.B. Zytomegalie-Virus IE-Promotor (CMV-IE). Weiterhin wurden auch solche Promotoren durch die Fusion von Nukleinsäuresequenzen konstruiert, die zusätzlich bei Virusbefall eine verstärkte Expression induzieren sollten. Insbesondere ist ein Bestandteil dieser Hybridpromotoren das CMV-IE-Metallothionein Promotorfragment und das HIV-1 LTR Nukleinsäurefragment der Nukleotide 289 bis 474 oder 289 bis 532 (Nukleotid-Numerierung: UWGCG GENEMBL DATA BANK File: HIVHXB2CG. VIRAL vom 25. Sept. 1987), das zu verstärkter, gegebenenfalls transaktivierbarer, zusätzlicher Promotoraktivität beiträgt.

### Zu den Antisense-Nukleinsäurefragmenten

Weiterhin enthalten die Vektorkonstrukte unter der Kontrolle der beschriebenen Promotoren subgenomische, provirale DNA Fragmente in Antisense-Orientierung, das heißt, der (+)-Strang der DNA wird in 3' → 5' Richtung transkribiert.

Es handelt sich aus Sicherheitsgründen nicht um ein komplettes, provirales genomisches DNA Fragment eines Retrovirus. Aber es handelt sich insbesondere um längere subgenomische, provirale DNA Fragmente und weniger um kurze, subgenische provirale DNA Fragmente, weil bei längeren RNA Transkripten eher die Wahrscheinlichkeit des RNA-Spleißens erfolgt und auf diese Weise von den erfindungsgemäßen proviralen DNA Fragmenten gegebenenfalls mehrere Antisense-RNA Transkripte zu erwarten sind und in den genannten Beispielen auch tatsächlich gefunden wurden. (Die Art und Anzahl der zu erwartenden, vollständig prozessierten Antisense-mRNA Transkripte ist für die verwendeten HIV-Sequenzen in Antisense-Orientierung nicht vorhersagbar, weil es für das Spleißen von mRNA keine hinreichend genauen Konsensussequenzen für eine schlüssige Voraussage gibt.)

Mit steigender Zahl von Antisense-RNA Transkripten mit ihren inherenten Eigenschaften, wie z.B. Export aus dem Nukleus oder Stabilität im Cytoplasma, steigt auch die Wahrscheinlichkeit, ein potent antiviral wirksames Antisense-RNA Transkript zu erzeugen.

Die Erfindung betrifft weiterhin Expressionsvektorkonstrukte, die unter der Kontrolle eines starken Promotors ein fusioniertes Genfragment zur Transkription von Antisense-RNA enthalten. Dabei besteht das fusionierte Genfragment aus subgenomischen DNA-Fragmenten aus zwei verschiedenen oder mehreren verschiedenen pathogenen Viren, z.B. aus HIV-1 und HIV-2. Mit solchen fusionierten Genfragmenten, die ein RNA-Transkript kodieren, das nacheinander mehrere Antisense-RNA Sequenzbereiche enthält, die komplementär zur mRNA-Sequenzbereichen aus verschiedenen Viren sind, lassen sich Expressionsvektoren mit breiterer antiviraler Wirkung konstruieren.

Die Gültigkeit dieses Prinzips läßt sich an den Beispielen 1, 4, 5, 10 und 12 belegen. Zum Beispiel enthalten die Expressionsvektorkonstrukte KTX 11, pSXK1 und pSXK2 Antisense-DNA Fragmente aus HIV-1 LAV/BRU, die ihrerseits kürzere Abschnitte enthalten, die auch nur mRNA von HIV-2 D194 komplementär sind (z.B. Nukleotide 620-660 oder 837-899 oder 4868-5044).

Auf diese Weise ist die Antisense-RNA aus KTX11, pSXK1 und pSXK2 auch antiviral gegen HIV-2.

Man kann nun die Antisense-RNA kodierende Sequenz dieser Vektoren zusätzlich mit Fragmenten proviraler DNA in Antisense-Orientierung aus anderen Viren (z.B. HIV-2 Isolate, andere HIV-1 Isolate) fusionieren, so daß abschnittsweise aufgrund optimierter Komplementarität der Antisense-RNA zur Sequenz der anderen Viren, gleichzeitig eine bessere oder vollständige Hemmung verschiedener Viren mit einem Expressionsvektorkonstrukt erreicht werden kann.

Die Terminierung der Antisense-RNA Transkripte erfolgt mit dem Polyadenylierungssignal (z.B. aus SV 40 oder aus dem bovinen Wachstumshormon), um eine hochstabile mRNA zu generieren. Weiterhin enthalten die Vektoren das vollständige Operon zur Expression eines geeigneten Selektionsmarkers. In den aufgeführten Beispielen wird eine Antibiotikaresistenz TNR 5 neo (Neomycin, Geneticin, G 418, Kanamycin) genutzt, um Zellen selektionieren zu können, die nach der Transfektion das Genkonstrukt für die Antisense-RNA Expression enthalten.

Dieser Selektionsmarker hat sich für menschliche hämatopoetische Zellen bewährt. Andere Selektionsmarker, wie z.B. Puromycin oder Hygromycin oder Methotrexate in Kombination mit der Transfektion des Gens für die Dihydrofolsäure-Reduktase, könnten auch verwendet werden.

Besonders geeignet als Antisense-Nukleotidsequenzen sind solche aus dem POL/VIF Genbereich von HIV zwischen den Nukleotiden 2100-5800. Insbesondere Antisense-Nukleotid-sequenzen der Nukleotide 5786→4158 haben eine herausragende antivirale Wirkung gezeigt. Es können auch andere Fragmente aus diesem Genbereich verwendet werden, z.B. 5746→4648 oder 4647→4157 oder 5880→3880 oder 5850→4158 oder 5150→3200.

Für den Nachweis der antiviralen Wirkung der Antisense-RNA z.B. gegen HIV-1 wurden menschliche Zellinien, die mit HIV-1 infizierbar sind, mit den zuvorgenannten Expressionsvektorkonstrukten transfiziert. Es sollte an einer Reihe von Zelltypen menschlicher hämatopoetischer Zellen nachgewiesen werden, daß die endogene Antisense-RNA Expression zu einer Hemmung der Virusreplikation in Zellen führt, die insbesondere an der zellulären Immunreaktion beteiligt sind. Dieser Abbau von Zellen des Immunsystems in der Endphase der AIDS Erkrankung steht in ursächlichem Zusammenhang mit der erhöhten Häufigkeit opportunistischer Infektionen, die dann oft tödlich verlaufen. Deshalb war es das Ziel der Arbeiten in hämatopoetischen Zellen, und hier zunächst an permanenten Zellinien wie z.B. HUT 78, MOLT 4, U 937, Jurkat, CEM-CM3 (alle American Type Culture Collection ATCC, Bethesda, Maryland, USA), nach der Vektortransfektion, Klonierung der Zellinien und genetischer Charakterisierung, in Infektionsversuchen mit geeigneten HIV-1 und HIV-2 Isolaten, die "Virusresistenz" dieser transfizierten Zellinien nachzuweisen.

Der Nachweis der Expression antiviral wirksamer Antisense-RNA in diesen Zellen sollte die Brauchbarkeit der Expressionskonstrukte in blutbildenden Zellen und letztlich auch deren Verwendung zur Transformation von Zellen des blutbildenden Knochenmarkgewebes für therapeutische Anwendungen aufzeigen. Das Einbringen von Genen in periphere Blutlymphozyten und Knochenmarkzellen zu therapeutischen Zwecken wurde an anderer Stelle bereits beschrieben [K. W. Culver et al., Transplant Proc. 23, 170 - 177 (1991)] und sollte für die hier genannten Antisense-RNA Expressionskonstrukte prinzipiell übertragbar sein.

### Beispiele

### Beispiel 1

### Expressionsvektorkonstrukt KTX 11

(Im folgenden bezieht sich alle Nukleotidnumerierung auf Human immunodeficiency virus type 1 (HXB2) Sequenz, UWGCG, GENEMBL DATA BANK, File: HIVHXB2CG.VIRAL vom 25-Sep-1987, wenn nicht anders angegeben.)

Zur endogenen Expression von antiviral wirksamer Antisense-RNA nach der Transfektion von HIV-1 infizierbaren Zellen wurde folgender Expressionsvektor konstruiert:
Der murine Metallothionein I Promotor [1600 Basenpaare EcoRI - Bgl II Fragment (N. Glanville et al., Nature 292, 267 - 269 (1982))] wurde mit der Sal I Schnittstelle (Nukleotid 5786) aus dem 3'-Bereich der proviralen HIV-1 DNA-Sequenz nach Ligation eines Bgl II-Xho I Adapters ligiert, wobei die Bgl II, Xho I und Sal I Schnittstellen umgewandelt wurden.

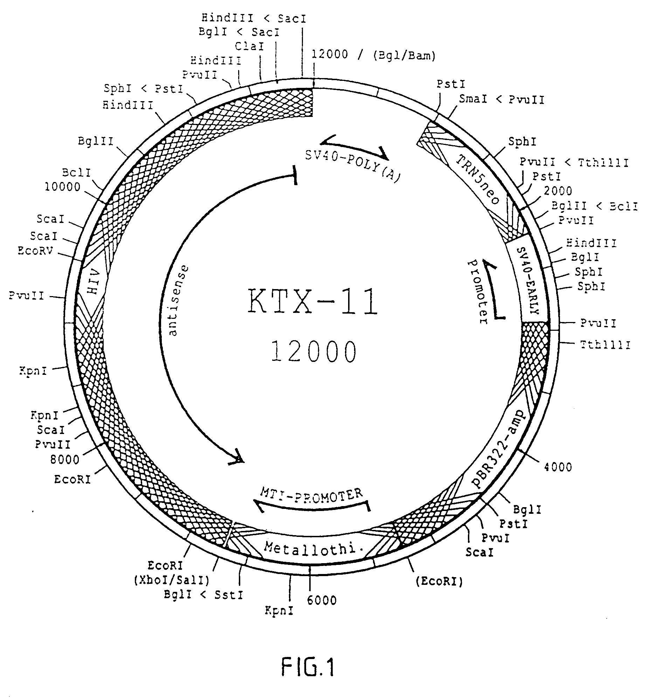

Die Bgl II Schnittstelle (Nukleotid 474) aus dem 5'-Bereich der proviralen HIV-1 DNA wurde mit einer Bam HI Schnittstelle ligiert, an die sich die Sequenz des SV 40 Polyadenylierungssignals anschließt (Nukleotide 2564 bis 4713 mit deletierten Sequenzen aus UWGCG GenEMBL File SV 40XX). Dieselbe Polyadenylierungssequenz aus SV 40 wird in 3' → 5' Richtung für die Terminierung des TRN5NEO [Selektionsmarker Neomycin (Phosphotransacetylase), Nukleotide 1 - 1286 TRN5NEO BACTERIA, UW GCG GenEMBL] verwendet. Die Neomycingenexpression wird vom SV 40 Early Promotor (Nukleotide 5171 - 371, UWGCG GenEMBL SV 40XX) angetrieben. Ansonsten enthält der Vektor noch pBR 322 DNA (Nukleotide 2067 - 4363, UWGCG GenEMBL File PBR 322) mit bakteriellem Origin und Ampicillin Resistenzgen, um ihn als Shuttle-Vektor für E. coli und tierische Zellen verwenden zu können. Eine Genkarte dieses Vektors KTX 11 ist in Abbildung 1 dargestellt.

### Beispiel 2

### Expressionsvektorkonstrukt KTX 12

Der HIV-1 Antisense RNA Expressionsvektor KTX 12 ist in gleicher Weise wie der Vektor KTX 11 (Beispiel 1) aufgebaut, jedoch enthält er einen induzierbaren Hybridpromotor und der HIV-1 Template DNA-Strang erstreckt sich in 3' → 5' Orientierung hinter diesem Hybridpromotor von den Nukleotiden 7514 → 474.

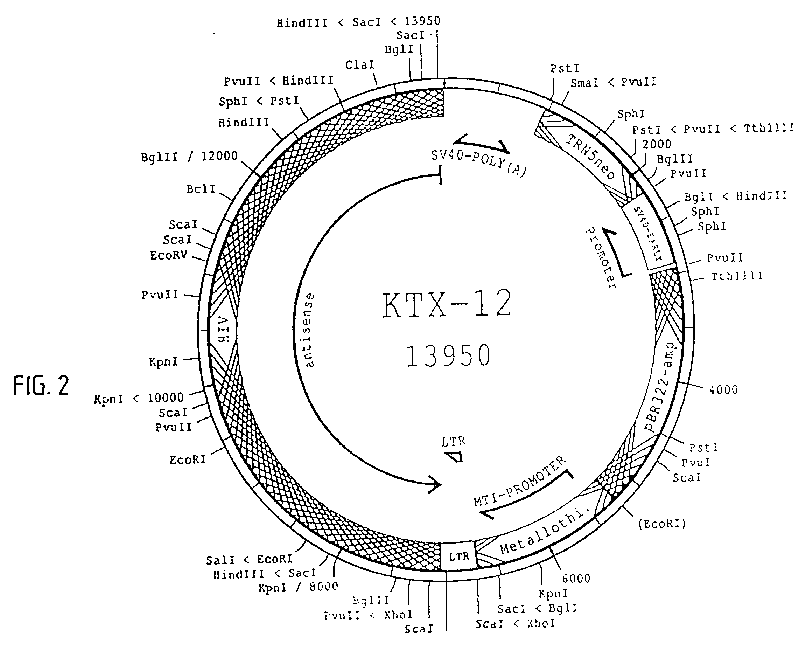

Für den Hybridpromotor wurde an dem Metallthionein-Promotor Nukleotid 368 (UW GCG File MUSMETI.RO) das HIV-1 LTR Promotor Fragment Nukleotid 289 bis 532 fusioniert. Eine Restriktionskarte dieses Vektors KTX 12 ist in Abbildung 2 dargestellt.

### Beispiel 3

### Expressionsvektorkonstrukt KTX 15

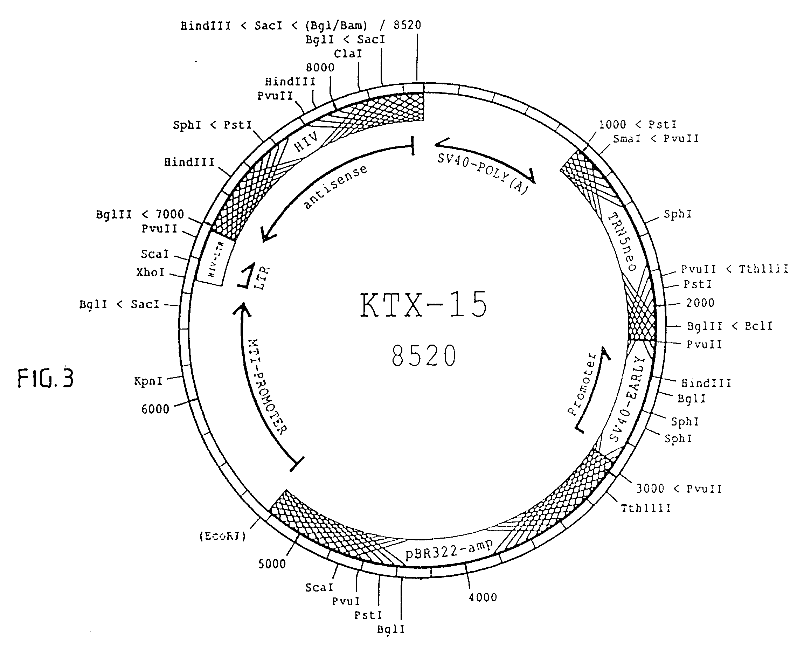

Der HIV-1 Antisense RNA Expressionsvektor KTX 15 ist in ähnlicher Weise wie der Vektor KTX 12 (Beispiel 2) aufgebaut, jedoch ist das DNA Fragment des HIV 1 Template Stranges (in 3' → 5' Orientierung) wesentlich verkürzt, es reicht von Nukleotid 2096 bis zum Nukleotid 474 und umfaßt damit die GAG-Sequenz, die Leader-Sequenz, die U5-Sequenz und einen Teil der R-Sequenz. Eine weitere wichtige Eigenschaft des Vektors KTX 15 ist die Deletion der TAR-Sequenz mit der Deletion der Nukleotide 474 - 532 im HIV-LTR Hybridpromotor. Dadurch sollte der Hybridpromotor immer noch bei HIV-Infektion stimulierbar sein, aber die TAR-codierte Termination der Transkription [S. Y. Kao et al., Nature 330, 489 (1987)] ohne Gegenwart des HIV-1 TAT Proteins sollte unterbunden sein. Dadurch zeichnet sich dieser Hybridpromotor durch eine besonders hohe konstitutive Transkription unter der Kontrolle einer HIV-1 LTR Promotorsequenz aus. Eine Restriktionskarte des Vektors KTX 15 ist in Abbildung 3 dargestellt.

### Beispiel 4

### Expressionsvektorkonstrukt pSXK 1

Der HIV-1 Antisense-RNA Expressionsvektor pSXK 1 ist hergestellt worden, um die Expression von Antisense-RNA unter der Kontrolle eines anderen, stark konstitutiv exprimierenden Promotors, hier am Beispiel des Cytomegalieviruspromotors, zu zeigen. In die Hind III Restriktionsstelle am Nukleotid 891 (Vektor pRc/CMV, Katalog-Nr. V 750-20 Fa. Invitrogen, San Diego, CA 92121, USA) wurde das HIV-1 Genfragment Nukleotid 681 - 3825 in 3' → 5' Orientierung insertiert. Das Polyadenylierungssignal entstammt dem Rinderwachstumshormon (BGH)-Gen.

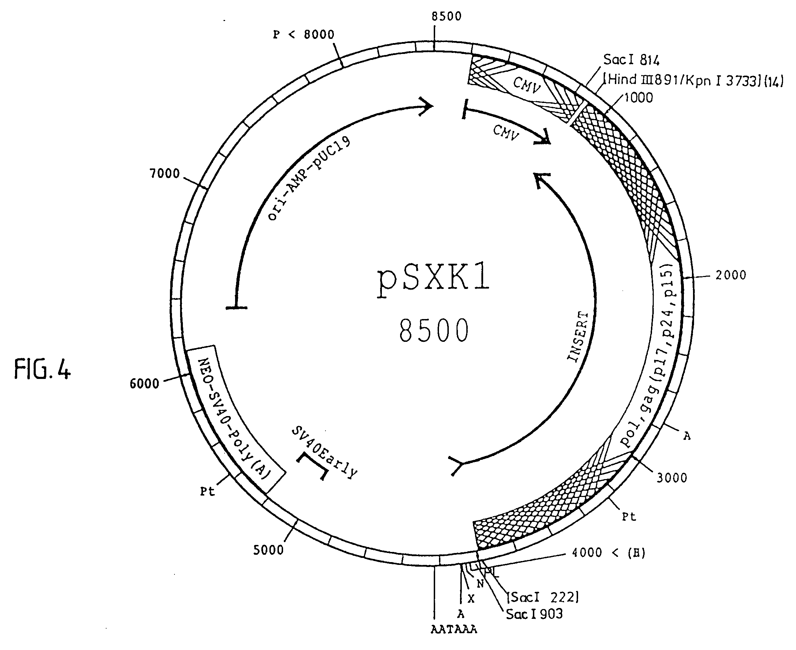

Ansonsten ist der Vektor mit dem Neomycin-Selektionsmarker und einem pBR322 DNA-Fragment zur Replikation und Selektion in E.coli als Shuttle-Vektor in gleicher Weise aufgebaut wie der Vektor KTX 11 (Beispiel 1). Eine Restriktionskarte des Vektors pSXK 1 ist in Abbildung 4 dargestellt.

### Beispiel 5

### Expressionsvektorkonstrukt pSXK 2

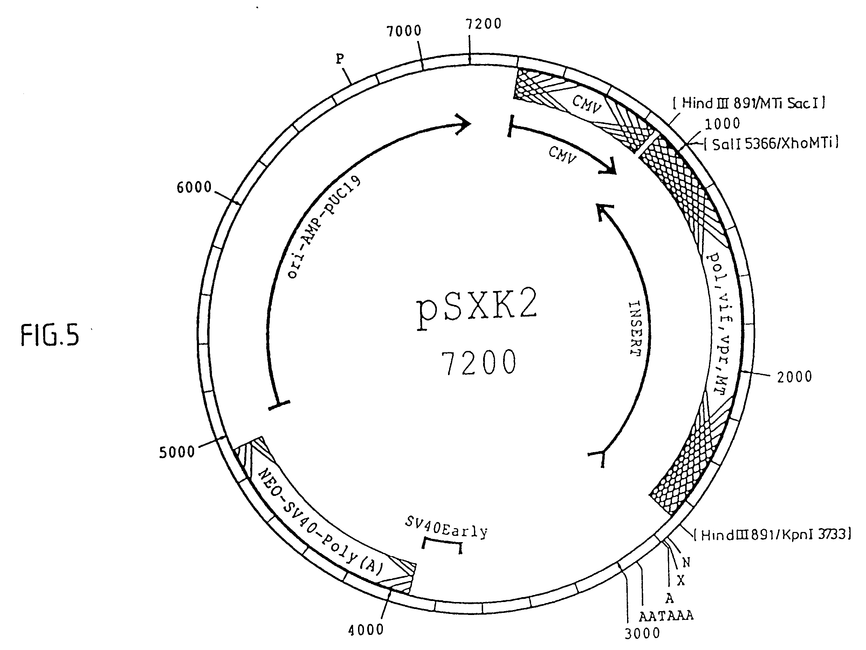

Der HIV-1 Antisense-RNA Expressionsvektor pSXK 2 unterscheidet sich von dem Vektor pSXK 1 (Beispiel 4) dadurch, daß er fusionierte Promotorsequenzen bestehend aus den Nukleotiden 209 - 865 des CMV-Promotors (siehe Beispiel 4), fusioniert mit den Nukleotiden 150 - 369 Metallothionein-Promotor (UWGCG GenEMBL File MUSMETI.RO) aufweist und damit ebenfalls einen Hybridpromotor darstellt. Außerdem wurde die provirale DNA-Sequenz der Nukleotide 4158 - 5786 in 3' → 5' Orientierung als Template für die Antisense-RNA Expression unter der Kontrolle des CMV/Metallothionin Hybridpromotors insertiert. Die Restriktionskarte des Vektors pSXK 2 ist in der Abbildung 5 wiedergegeben.

### Beispiel 6

### Expressionsvektorkonstrukte pHTT 1, 4, 6, 9, 10, 12 und 15

Die HIV-1 Antisense RNA Expressionsvektoren pHTT 1, 4, 6, 9, 10, 12 und 15 sind in ähnlicher Weise wie Vektor pSXK 1 (Beispiel 4, Abb. 4) hergestellt worden. Alle die in den pHTT-Vektoren eingesetzten HIV-1 Genfragmente wurden in 3'-5' Orientierung in den Vektor pRc/CMV (Firma Invitrogen, San Diego, CA 92121, Katalog-Nr. V 750-20, 1991) unter Kontrolle des konstitutiv exprimierenden Cytomegalieviruspromotor kloniert.

Nachfolgend werden die pHTT Vektoren spezifiziert:
(a) pHTT 1: Das HIV-1 Genfragment (Nukleotid 4648 - 5746) wurde in die Not I Schnittstelle (Nukleotid 966) des pRc/CMV Vektors eingebaut.
(b) pHTT 4: Das HIV-1 Genfragment (Nukleotid 1635 - 2369) wurde in die Xba I Schnittstelle (Nukleotid 985) des pRc/CMV Vektors eingebaut.
(c) pHTT 6: Das HIV-1 Genfragment (Nukleotid 3003 - 3227) wurde in die Xba I Schnittstelle wie unter (b) beschrieben eingebaut.
(d) pHTT 9: Das HIV-1 Genfragment (Nukleotid 678 - 2099) wurde in die Xba I Schnittstelle wie unter (b) beschrieben eingebaut.
(e) pHTT 10: Das HIV-1 Genfragment (Nukleotid 2099 - 3829) wurde in die Xba I Schnittstelle wie unter (b) beschrieben eingebaut.
(f) pHTT 12: Das HIV-1 Genfragment (Nukleotid 4157 - 4647) wurde in die Xba I Schnittstelle wie unter (b) beschrieben eingebaut.
(g) pHTT 15: Das HIV-1 Genfragment (Nukleotid 3830 - 4157) wurde in die Xba I Schnittstelle wie unter (b) beschrieben eingebaut.

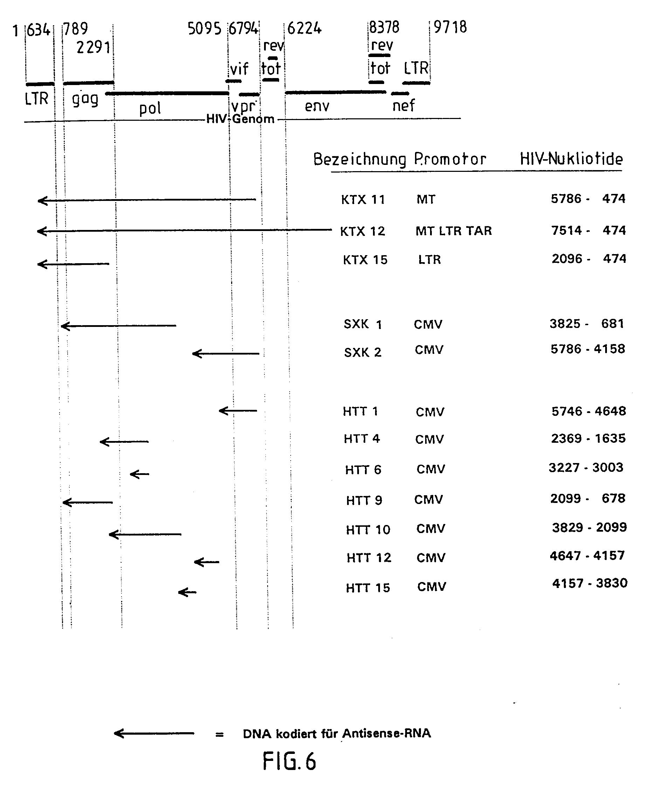

Ein Übersichtsdiagramm der HIV-1 Genfragmente, die in Antisense-Orientierung (3'→5') in den Vektoren der Beispiele 1 bis 6 enthalten sind, ist mit der Abbildung 6 wiedergegeben.

### Beispiel 7

### Transfektion und Klonierung menschlicher Lymphozyten mit Antisense-RNA Expressionsvektoren

Als Beispiele von transfizierten menschlichen hämatopoetischen Zellinien, die Virusresistenz durch endogen exprimierte Antisense-RNA gegen HIV-1 aufweisen, wurden die nicht adhärent wachsende, monozytenähnliche Zellinie U 937 und die nicht adhärent wachsende T-Lymphozyten Zellinie HUT 78 ausgewählt. Beide Zellinien wurden von der American Type Culture Collection (ATCC), 12301 Parklawn Drive, Rockville, MD 20852 - 1776, USA, erhalten.

Die Zellinien wurden in dem von der ATCC empfohlenen Medium kultiviert. Die Vektortransfektion erfolgte durch verschiedene Verfahren, wobei die Elektroporation die besten Erfolge zeigte:
1. Calciumphosphat Methode:
   Variiert nach Wigler et al., 1977, Cell 11, 223 Die Transfektion wird mit Ca-Präzipitaten der DNA durchgeführt. Variierte Mengen von 0,05 bis 20 µg DNA wurden in 0,25 ml 250 mM CaCl₂ gelöst und langsam zu 0,25 ml 2 x HEBS-Puffer (1,5 mM Na₂HPO₄, 50 mM Hepes, pH 7,0) pipettiert. Die Transfektion erfolgte durch Zugabe des gesamten Präzipitat-Ansatzes in 10 ml Zell-Kultur. 15 Stunden später wurde das Medium abgesaugt. Danach wurden die Zellen mit DMSO geschockt.
   Für eine 10 ml Kultur wurden 3 ml einer 25 %igen DMSO-Lösung in sterilem PBS hinzugegeben. Nach 4 Min. wurde abgesaugt und mit 5 ml PBS gewaschen. Anschließend wurden die Zellen in 10 ml DMEM-Medium aufgenommen.
2. DEAE-Dextran
   Am Tage vor der Transfektion wurden Zellen zu einer Dichte von 3-4 x 10⁵ pro ml Kulturmedium umgesetzt. 2-4 h vor der Transfektion wurden die Zellen zentrifugiert und in neuem Medium aufgenommen. 0,5 bis 20 µg DNA wurden mit 1070 µl PBS gemischt; dazu wurden 120 µl einer 10 mg/ml DEAE-Dextranlösung gegeben (Transfektionslösung). Vor der Transfektion wurden 40 ml der Zellen zentrifugiert, in 10 ml PBS aufgenommen und nochmals zentrifugiert.
   Das Zellpellet wurde in 1,2 ml PBS-DEAE-Dextranlösung suspendiert und 30 Min, inkubiert. Danach wurden die Zellen durch Zugabe von 0,8 ml kalter DMSO-Lösung (25 %ig in TBS(25 mM Tris-HCl, 137 mM NaCl, 0,7 mM CaCl₂, 0,5 mM MgCl₂, 5 mM KCl, 0,6 mM Na₂HPO₄, pH 7,4)) geschockt. Nach 3 Min. wurde 10 ml TBS zugesetzt und zentrifugiert. Die Zellen wurden in RPMI Wachstumsmedium gewaschen und in 40 ml des gleichen Mediums aufgenommen.
3. Fusion (Sanari-Goldin Methode)
   Logarithmisch wachsende Zellen (1 x 10⁷ Zellen/ml) wurden zentrifugiert und einmal mit RPMI-Medium gewaschen. Zu dem Zellpellet wurden 2 ml Protoplastensuspension von E.coli (2 x 10³ Zellen/ml in 10 % Sucrose und 10 mM MgCl₂) zugesetzt. Die Protoplasten mit dem zu transfizierenden Plasmid wurden nach Sanari-Goldin et al. hergestellt. Nach 8 Min. Inkubation wurde zentrifugiert und vorsichtig innerhalb von 2 Min, PEG-Lösung (Polyethylenglycol 400; 45 %ige Lösung in RPMI) zugesetzt. Nach 1 Min. Inkubation wurde innerhalb von 7 Min. 10 ml RPMI-Medium ohne FCS zugesetzt. Die Zellen wurden zentrifugiert, in RPMI-Medium gewaschen und in 10 ml aufgenommen.
4. Elektroporation
   Die Elektroporation (EP) wurde mit dem Apparat Gene Pulser Apparatus and Capacitance Extender der Fa. Bio Rad durchgeführt. Logarithmisch gewachsene Zellen wurden zu einer Zelldichte von 1 x 10⁷ Zellen/ml eingestellt, 1 x in PBS gewaschen und in RPMI Medium ohne FCS mit 10 mM Dextrose und 0,1 mM Dithiothreitol resuspendiert.
   0,4 ml der Zellsuspension wurden in eine EP Küvette gegeben und 10 - 20 µg DNA zugesetzt. Die DNA wurde zu 1 µg DNA pro 1 µl TE gelöst.
   Die Elektroporationsbedingungen wurden wie folgt variiert:
   Kapazität: 260-960 µF; Spannung 150-300 V; Elektrodenabstand 0,2/0,4 cm; Widerstand 100 - ∞ Ohm.
   Vor und nach der Elektroporation wurden die Zellen 10 Min. bei Raumtemperatur inkubiert, danach in 10 ml Wachstumsmedium + 10 % FCS umgesetzt.

### Selektion von Transfektanten;

Die Selektion von Transfektanten wurde in jedem Fall über die Resistenz gegenüber G 418 durchgeführt.
1. Selektion in viskosen Medien.
   Die transfizierten Zellen wurden zunächst in G 418-haltigem Wachstumsmedium so lange vorinkubiert, bis kein Wachstum mehr feststellbar war. Danach wurde in viskosem Medium ausgesät. Zur Verfestigung des Mediums diente entweder Agar (Gibco) oder Methylzellulose. Die vorselektionierten Zellen wurden in eine Gewebekulturplatte (10 cm) ausgesät, dazu wurden 10 ml Selektionsmedium (RPMI plus 1 mg/ml G 418) und 2 ml einer 2 %igen verflüssigten Agarlösung gegeben. Nach dem Erkalten des Agars wurde im Brutschrank bei 37°C inkubiert. In einer Versuchsvariante wurde der Agar durch Methylzellulose ersetzt.
2. Selektion mit anschließender Klonierung in Transwell cell culture chamber (Transwell TM, Fa. Costar)
   Zellen wurden 48 Stunden nach der Transfektion zu 1 x 10⁵ Zellen pro 0,2 ml Well einer 96 Well-Microtiterplatte ausgesät. G 418 wurde zu einer Konzentration von 0,7 mg/ml für U 937 und 1,0 mg/ml für HUT 78 zugesetzt. 1/2 Volumen des Wells wurde, sobald das Medium verbraucht war, mit neuem Medium ausgetauscht; bei U 937 wurde dann nur noch 0,5 mg/ml G 418 zugesetzt.
   Es wurde solange inkubiert, bis in den einzelnen Wells resistente Zellen anwuchsen. Diese Mischklone wurden auf ein größeres Volumen expandiert und anschließend kloniert. Hierzu wurde eine spezielle Wachstumskammer der Fa. Costar (Transwell TM, Katalog-Nr. 3425, 205 Broadway, Cambridge, MA 02139, USA) eingesetzt.

Die Zellen wurden in geringer Zelldichte (50 bis 200 Zellen pro 6 ml) in Weichagar mit G 418 (siehe Selektionierung in viskosem Medium) ausgesät und 6 ml in eine zentrale Kammer (Upper compartment) gegeben. Die Kammer ist durch eine Membran von einer sehr dicht wachsenden flüssigen Feederkultur von U 937 bzw. HUT 78 (200 000 Zellen /ml) abgetrennt (Lower compartment). Das flüssige Medium wird nach Verbrauch teilweise gewechselt, um weiteres Zellwachstum zu ermöglichen. Sobald G 418 resistente Kolonien im Soft-Agar erschienen, wurden sie mit einer Pasteurpipette isoliert und expandiert.

Auf diese Weise wurden mit den Vektoren KTX 11, KTX 12, KTX 15, pSXK 1 und pSXK 2 jeweils etwa zehn unabhängige, transfizierte Zellinien für U 937 und HUT 78 kloniert und für den Nachweis der Antisense-RNA Expression und der HIV-1 Resistenz weiter bearbeitet Weiterhin wurden mit den Vektoren pHTT1, pHTT4, pHTT9 und pHTT10 HUT-78-Zellinien generiert und in HIV-Infektionstests geprüft.

### Beispiel 8

### Transformation von Knochenmarkzellen mit dem Expressionsvektor KTX 15

Für grundlegende Beispielversuche zum Nachweis der Transformation primärer Knochenmarkzellen wurde am Maus-Modell wie folgt vorgegangen:
Primäre Knochenmarkzellen wurden gewonnen aus den Oberschenkelknochen von sechs bis zehn Wochen alten C57 Mäusen. Die Zellen wurden in McCoy Medium (Fa. Flow) aufgenommen und mit Interleukin 3 und GM-CSF (Granulozyten-Monozyten colony stimulating factor; Fa. Genzyme) in einer Konzentration von jeweils 2,5 µg/ml und Agar (Bacto Agar, Fa. Difco; Endkonzentration 0,3 %) vermischt und in einer Konzentration von 1x10⁵ Zellen pro ml in Gegenwart oder Abwesenheit vom toxischen Antibiotikum G418 (Neomycin) in Petrischalen ausplattiert. Eine Transfektion wurde direkt nach der Entnahme der Knochenmarkzellen vor dem Ausplattieren in Agar durchgeführt. Anschließend wurden die Zellen mehrere Tage bei 37°C im Brutschrank bei 7,5 % CO₂ kultiviert. Im Mikroskop wurde dann nach verschiedenen Zeitintervallen jeweils die Anzahl der transformierten Kolonien (mindestens 50 Zellen) bzw. Cluster (10 bis 50 Zellen) ausgezählt. Unter den gegebenen Versuchsbedingungen waren die meisten Kolonien gemischte Granulozyten/Monozyten oder Granulozyten Kolonien, selten waren erythroide Bursts, einmal wurden Megakaryozyten beobachtet Die Identifizierung der Zelltypen in den Kolonien erfolgte durch May-Grünewald Färbung.

Die Transfektionsbedingungen wurden optimiert. Erfolgreiche Transfektionen von primären Knochenmarkzellen wurden mit folgendem Ansatz erhalten:
800 µl Knochenmarkzellsuspension (5·10⁶ Zellen in Elektroporationsmedium wie in Beispiel 7 beschrieben), 5 µg KTX15, Elektroporation bei 300 V, 100 Ω, 250 µF, 6 ms.

Nach der Elektroporation (Gene Pulser Apparatus and Capacitance Extender der Fa. Bio Rad) wurden die Zellen im oben genannten Kulturmedium in Gegenwart von 500 µg/ml G-418 (Neomycin) selektioniert. Die Resistenz gegen G-418 zeigte die erfolgreiche Transfektion propagierbarer primärer Knochenmarkzellen an.

### Beispiel 9

### Nachweis der Integration intakter Expressionsvektorkonstrukte und Nachweis der Antisense-RNA Expression

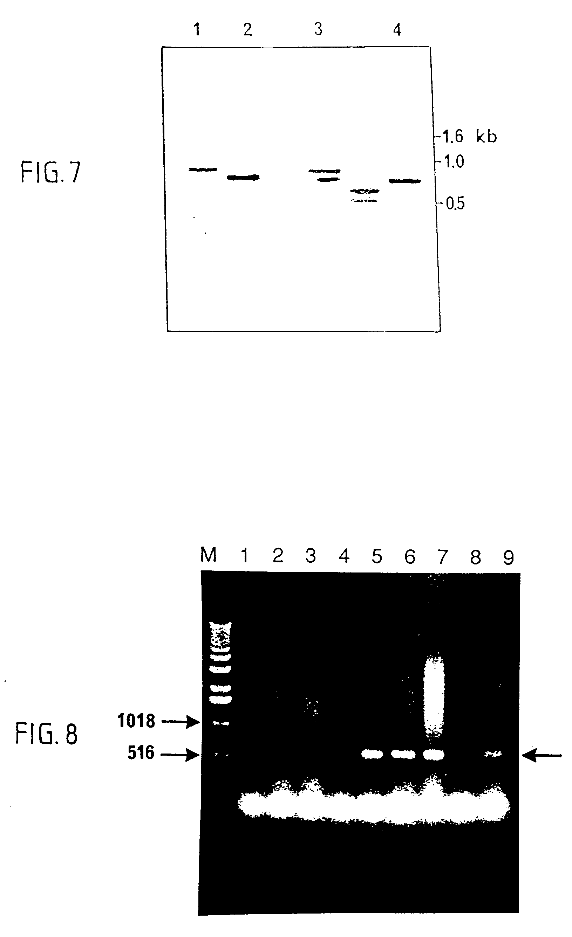

Durch Cäsiumchlorid Dichte-Gradientenzentrifugation wurden nach einem Aufschluß der transfizierten U 937 und HUT 78 Zellinien die DNA und RNA aus diesen Zellinien mit Standardmethoden gewonnen [L. G. Davis et al., Basic Methods in Molecular Biology, Elsevier, New York (1986)]. Der Nachweis der Integration intakter Vektorkonstrukte zur Antisense-RNA Expression wurde durch Polymerase-Chain Reaktion [PCR Technology Principles and Application for DNA Amplification, Ed. Henry A. Erlich, M. Stockton Press, New York (1989)] mit Oligonukleotid Primern durchgeführt, die überlappende DNA Fragmente von Promotorsequenz, Insertsequenz und Terminierungssequenz amplifizierten. Anschließend wurde die amplifizierte DNA durch Southern Blot Analyse (Davis et al., s.o.) mit Insert DNA Gensonden identifiziert. Beispielhafte Analysenergebnisse sind in den Abbildungen 7, 8, 9 dargestellt. Für die Vektoren KTX 11, KTX 12 und KTX 15 wurden z.B. die flankierenden Primer 5'-CAA ACC CTT TGC GCC CG-3' oder 5'-ACT CGT CCA ACG ACT AT-3' aus der Promotor-Sequenz und 5'-TTT TTT CAC TGC ATT CTA CTT-3' aus der Polyadenylierungssequenz verwendet Für die Expressionsvektoren mit dem pRc-CMV-Vektor wurden z.B. die flankierenden Primer 5'-CTT TCC AAA ATG TCG TAA CAA CTCC-3' für die Promotorsequenz und 5'-ATT TAG GTG ACA CTA TAG AAT-3' für die Terminationssequenz verwendet Im Insert (HIV-Sequenz) wurden Primersequenzen gewählt, die PCR-Produkte je nach Wahl der Primerpaare von ca. 500 Basenpaaren bis 2.100 Basenpaaren ergaben.

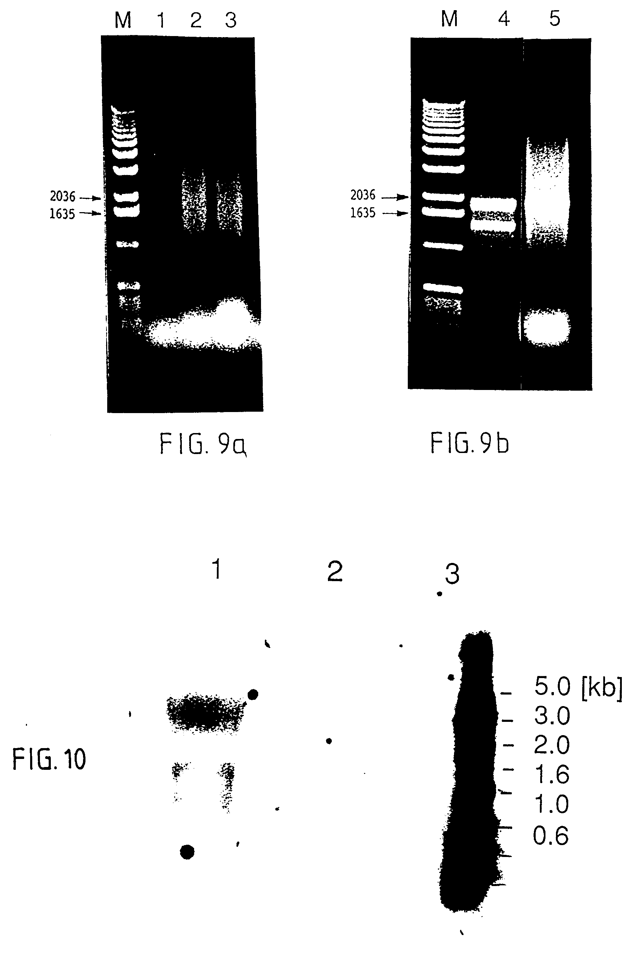

Der Nachweis der Antisense-RNA Expression wurde durch Northern Blot Analysen (Davis et al., s.o.) von RNA der klonierten, transfizierten Zellinien geführt. Ein Beispiel für ein Analysenergebnis zum Nachweis von HIV-1 Antisense-RNA in einer transfizierten U 937 Zellinie ist in Abbildung 10 dargestellt.

### Beispiel 10

### Nachweis der Virusresistenz von transfizierten, klonierten hämatopoetischen Zellininen

Klonierte, Antisense-RNA exprimierende Zellinien, die entsprechend der Beispiele 7 und 9 erhalten und charakterisiert wurden, wurden in vergleichenden Infektionsstudien mit HIV-1 [L. Ratner et al., Nature 313, 277 (1985)] oder HIV-2 D194 (H. Kühnel et al. Nucleic Acids Research (1990) 18, 6142) zusammen mit den Ausgangszellinien HUT 78 und U 937 als Kontrollen auf die Ausprägung der Virusresistenz untersucht.
1. Infektion von HUT 78 und U 937 Zellen mit HIV-1 (Kontrollen)
   Durchführung
   Je 10 ml Zellsuspension wurden 60 Min, mit unterschiedlichen Verdünnungsstufen von HUT 78/HIV-1 Kulturüberstand (ca, 10⁴ SFU/ml in PBL-Kulturen, RT-Aktivität der eingefrorenen Stammlösung 650 000 cpm/ml) versetzt (SFU = syncytia forming units).
   Die Zellen wurden anschließend abzentrifugiert und dreimal gewaschen, um ungebundene Viruspartikel und Virusproteine zu entfernen, Zuletzt wurden die Zellen in jeweils 10 ml Kulturmedium resuspendiert und im Brutschrank kultiviert.
   Alle 3 bis 4 Tage wurden 200 µl des zellfreien Kulturüberstands auf das Vorhandensein von HIV-Antigenen getestet Es wurde ein Antigen-Capture-Assay der Fa. Organon Technika verwendet.
   Zur Versorgung der Zellen wurden je 5 ml Zellsuspension entnommen und durch 5 ml Kulturmedium ersetzt.
2. Infektion von Antisense-RNA exprimierenden HUT 78- und U 937-Zellen mit HIV-1
   Durchführung
   Je 10 ml Zellsuspension (ca. 4 x 10⁵ Zellen/ml) wurden 60 Min, mit verdünntem Kulturüberstand von HUT 78/HIV-1 (ca. 10⁴ SFU/ml in PBL-Kulturen, RT-Aktivität der eingefrorenen Stammlösung 650 000 cpm/ml) versetzt; die Endverdünnung der Virusstammlösung betrug 1:1 000 für die HUT 78- und 1:50 für die U 937-Zellen, Die Zellen wurden anschließend abzentrifugiert und viermal vorsichtig gewaschen, um ungebundene Viruspartikel und Virusproteine zu entfernen, Zuletzt wurden die Zellen in jeweils 10 ml Kulturmedium (RPMI 1640 + 16 % FCS) resuspendiert und im Brutschrank 60 Tage lang kultiviert.
   Alle 3 bis 4 Tage wurden 160 µl des zellfreien Kulturüberstands auf das Vorhandensein von HIV-Antigenen getestet Es wurde eine Antigen-Capture-Assay der Fa. Organon Technika mit dem zugehörigen Mikrotiterplattenphotometer verwendet.
   Zur Versorgung der Zellen wurden alle 3 bis 4 Tage je 5 ml Zellsuspension entnommen und durch 5 ml frisches Kulturmedium ersetzt.

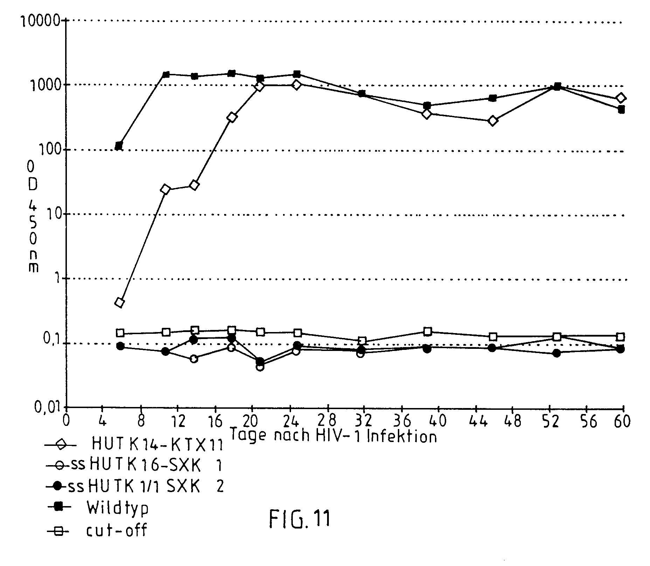

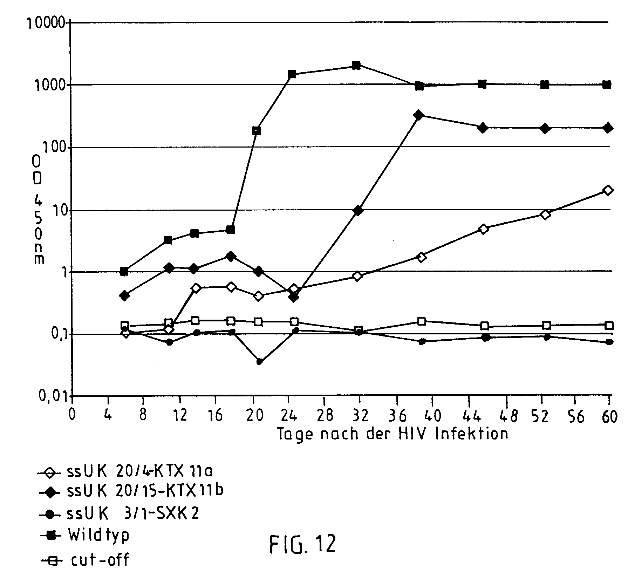

Als Ergebnis seien stellvertretend für andere HIV Antisense-RNA exprimierende Zellinien zwei Versuchsreihen mit drei HUT 78 Transfektanten (HUT K14-KTX11, SSHUTK16/2-SXK1, SSHUTK1/1-SXK2) (s.Abb. 11) und mit drei U 937 Transfektanten (ssUK 20/4-KTX11a, ssUK 3/1-SXK2 und ssUK 20/15-KTX11b) (Abb. 12) genannt, die sowohl bei der Messung der Reversen Transkriptase Aktivität (RT-Aktivität) als auch bei der Messung eines HIV Antigens in diesen Zellkulturen nach der Infektion im Verlauf der Inkubationszeit bis zu 60 Tagen keine Anzeichen der HIV-Vermehrung in diesen Antisense-RNA exprimierenden Zellinien mit den Expressionsvektoren pSXK1 und pSXK2 zeigen. Dagegen verläuft in der Kontrollzellinie die HIV Vermehrung deutlich meßbar ab. Außerdem ist ein verzögerter Anstieg der Virusreplikation in Transfektanten mit dem schwächer exprimierenden Expressionsvektor KTX11 festzustellen. Das Ergebnis ist in den Abbildungen 11 und 12 dargestellt.

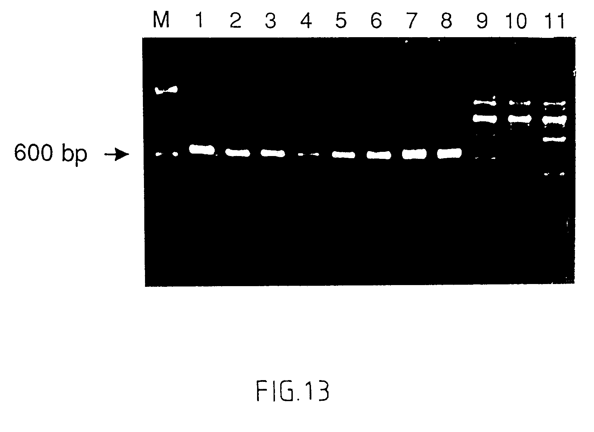

Zur Kontrolle der erfolgten HIV-1 Infektion in den Zellinien SSU K3/1-SXK2, SSHUT K16/2-SXK1 und SSHUT K1/1-SXK2, die nach der Inkubation von 60 Tagen keine Detektion von HIV-1 Antigen oder RT-Aktivität aufwiesen, wurde z.B. die positive Infektion durch eine PCR-Nachweisreaktion von HIV-1 TAT- und Envelopegensequenzen in den 60 Tage inkubierten Zellen von SSHUT K16/2-SXK1 und SSHUT K1/1-SXK2 nachgewiesen (Abb. 13). Dafür wurden die PCR-Primer 5'-ATG GAG CCA GTA GAT CCT-3' und 5'-TCT ACC ATG TCAT-3' zur Generierung eines 690 Basenpaare langen PCR-Fragments verwendet. Die Expressionsvektoren pSXK1 und pSXK2 enthalten diese Sequenzen nicht und können daher auch kein falsch-positives PCR-Produkt ergeben.

### Beispiel 11

### Kontrollversuche

Die Transfektion der Vektoren aus den Beispielen 1 bis 6 ohne die Inserts von proviraler DNA aus HIV-1 in Antisense-Orientierung führte in den Zellinien U 937 und HUT 78 nicht zu Zellinien, in denen die Virusreplikation gehemmt war. Kontrollversuche mit den Vektoren der Beispiele 1 bis 6, bei denen jedoch das provirale HIV-1 DNA Fragment in Sense-Orientierung insertiert ist, wurden nicht durchgeführt, weil die entsprechenden Sense-Transkripte regulatorische Proteine der HIV Replikation, wie z.B. TAT und REV, binden können und damit kompetitiv die Virusreplikation hemmen [G. J. Graham et. al., PNAS 87, 5817 (1990)] und daher als Kontrollexperimente ungeeignet erscheinen.

### Beispiel 12

### Infektion von Antisense-RNA exprimierenden HUT-78 und U937 Zellinien mit HIV-2

Infektionsversuche wurden mit dem Isolat HIV-2 D194 (H. Kühnel et al. Nucleic Acids Res. 18 (1990) 6142) durchgeführt. Die Virusstammsuspension mit einer RT-Aktivität von 2,14 · 10⁵ cpm/ml und einer Antigenkonzentration (1:1000 Verdünnung) von 0,249 OD₄₅₀ₙₘ (160 µl-Probe) wurde bei -80°C gelagert. Von dieser Virusstammsuspension wurden 50 µl für die Infektion von 2·10⁶ HUT 78 Zellen in 4 ml Kulturmedium eingesetzt und 3 Stunden inkubiert. U937 Zellen wurden mit 100 µl Virusstammsuspension in 4 ml Kulturmedium mit 2·10⁶ Zellen für 24 Stunden zur Infektion inkubiert. Anschließend wurden nicht gebundene Viruspartikel durch Waschen aus der Zellkultur entfernt.

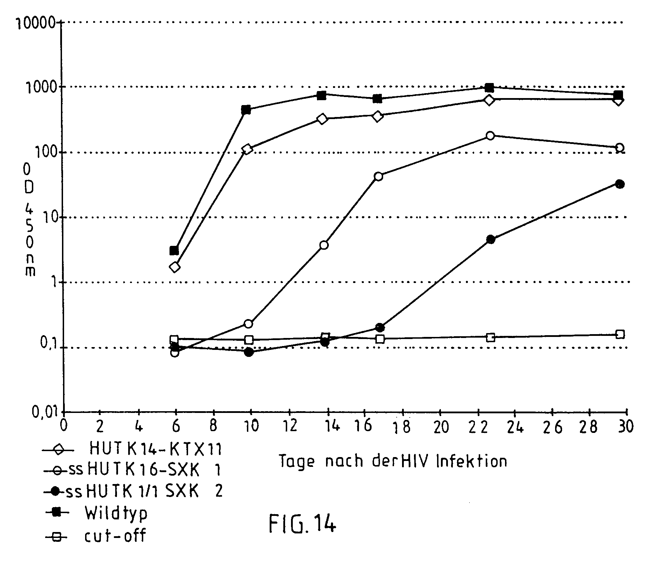

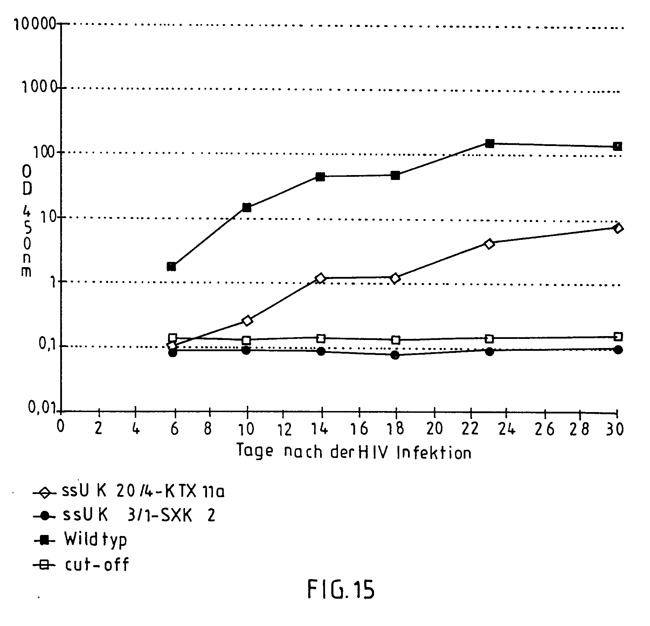

In Abständen von 3 bis 4 Tagen wurden die Zellkulturen versorgt und Zellkulturüberstand entnommen zur quantitativen HIV-Antigenbestimmung mit dem HIV-Antigen-Capture-Assay der Fa. Organon Technika in Verbindung mit dem zugehörigen Mikrotiterplattenphotometer. Der Verlauf der HIV-2 D194 Replikation in den HUT 78- und U937-Kontrollzellinien, die keine Antisense-RNA Expressionsvektoren enthalten im Vergleich zu den transfizierten Zellinien, die durch Antisense-RNA Expression eine verzögerte HIV-2 Vermehrung aufweisen, ist in den Abbildungen 14 und 15 dargestellt.

### Ergebnisse

Mit den Expressionsvektoren KTX 11, KTX 12, KTX 15, pSXK 1, pSXK 2, pHTT 1, pHTT 4, pHTT 6, pHTT 9, pHTT 10, pHTT 12 und pHTT 15 (Beispiele 1 bis 6) wurde in menschlichen Blutzellinien nach der Gentransfektion und der Klonierung transfizierter Zellinien (Beispiel 7) Antisense-RNA gegen HIV-1 endogen gebildet (Beispiel 9) und deren antivirale Wirkung nachgewiesen (Beispiele 10, 12).

Es ist überraschend, daß eine deutlich ausgeprägte Hemmung der Replikation des Retrovirus HIV-1 in den transfizierten Zellinien nachgewiesen wurde. Auch mit einem speziell an die transfizierten Zellinien adaptierten Virus HIV-1 konnte die Virusresistenz der Antisense-RNA exprimierenden Zellen in gleicher Weise gezeigt werden (Abb. 11,12). Ebenfalls wurde eine deutliche, antivirale Eigenschaft der Antisense-RNA gegen HIV-2 beobachtet (Abb. 14,15).

Diese Ergebnisse zeigen beispielhaft, daß geeignete Promotoren für die Expression von Antisense-RNA Molekülen und natürlich die Art der Antisense-RNA Sequenzen zu einer Resistenzausbildung genetisch veränderter Zellen gegen Retrovirusbefall führen. Im Beispiel 9 wurde gezeigt, daß aufgrund des bisher nicht bekannten RNA-Splicing von HIV-1 RNA Transkripten des (+)-Stranges der proviralen HIV-1 DNA in 3' → 5' Orientierung mehrere mRNA Moleküle entstehen, die zur antiviralen Wirkung führen.

Mit den experimentellen Arbeiten, die mit den Beispielen 1 bis 12 beschrieben wurden, wurde gezeigt, daß durch geeignete Expressionsvektorkonstrukte mit Antisense-RNA kodierenden DNA Fragmenten aus einem Retrovirus, antiretrovirale Eigenschaften in Zellen erzeugt werden können, die vor Retrovirusbefall schützen.

Aus den Abbildungen 11 bis 13 ist ersichtlich, daß die Zellinen SSHUT K16/2-SXK1 und SSHUT K1/1-SXK2 60 Tage nach der HIV-Infektion im Nachweis proviraler HIV-DNA mittels der PCR-Analytik positiv sind, aber der HIV Antigennachweis negativ ist, Dieses Ergebnis läßt den Schluß zu, daß nach der HIV-1 Infektion der Zellen zwar im ersten Schritt des HIV Replikationszyklus noch provirale DNA in den transfizierten HUT 78-Zellinien gebildet wird, aber die Transkription/Translation proviraler DNA durch die Antisense-RNA unterbunden wird und kein HIV-Protein für die Virusreplikation in nachweisbaren Mengen gebildet wird,

In der Zellinie SSU K3/1-SXK2 konnte 60 Tage nach Infektion mit HIV-1 in allen Testansätzen weder mit dem Antigentest noch mit der PCR-Analytik die Bildung von HIV-Proteinen bzw. provirale HIV-DNA nachgewiesen werden. Dieses Ergebnis ließe sich so deuten, daß die in der Zellinie SSU K3/1-SXK2 gebildete Antisense-RNA nicht nur den zweiten Schritt im HIV-Replikationszyklus (die Transkription und Translation proviraler DNA) unterbindet, sondern schon den ersten Schritt im Replikationszyklus hemmt:
Die Bildung proviraler DNA aus genomischer (+)-Strang RNA durch den Prozeß der reversen Transkription kann offensichtlich durch Antisense-RNA unterbunden werden. Diese Wirkung von Antisense-RNA wurde spekulativ angenommen, konnte jedoch bisher noch nicht gezeigt werden (R.Y.L. To and P.E. Neiman in Gene Regulation: Biology of Antisense RNA and DNA eds. R.P. Erickson and J.G. Izant, Raven Press Ltd. New York 1992), weil entsprechend leistungsfähige Expressionsvektorkonstrukte bisher nicht aufgefunden wurden.

### Legenden

### Abbildung 1

Expressionsvektor KTX 11 zur Expression von HIV-1 Antisense-RNA. Der Metallothionein Promotor (MTI-Promotor) treibt die Transkription von Antisense-RNA von einem proviralen HIV-1 DNA Fragment der Nukleotide 5786→474 [Numerierung: UWGCG GENEMBL DATA BANK File HIV HXB2CG.VIRAL]. Die Antisense-RNA Transkripte werden durch die SV 40 Polyadenylierungssequenz terminiert. Das Neomycin-Resistenzgen TRN5neo wird durch den SV 40 Early Promotor transkribiert. Der Shuttle-Vektor enthält noch das pBR322 Origin und das Ampicillin Gen (pBR 322-amp). Weitere Einzelheiten sind in Beispiel 1 beschrieben.

### Abbildung 2

Expressionsvektor KTX 12 zur Expression von HIV-1 Antisense-RNA unter der Kontrolle eines HIV-1 LTR Metallothioneinhybridpromotors. Einzelheiten zum Aufbau des Vektors sind in Beispiel 2 beschrieben.

### Abbildung 3

Expressionsvektor KTX 15 zur Expression von HIV-1 Antisense-RNA unter der Kontrolle eines HIV-1 LTR Metallothionein Hybridpromotors, bei dem die HIV-1 TAR Sequenz deletiert ist, um maximale Expression ohne Gegenwart des Transaktivatorproteins TAT zu erreichen. Einzelheiten zum Aufbau des Vektors sind in Beispiel 3 beschrieben,

### Abbildung 4

Expressionsvektor pSXK 1 zur Expression von HIV-1 Antisense-RNA unter der Kontrolle des Cytomegalievirus Promotors (CMV). Einzelheiten zum Aufbau des Vektors sind in Beispiel 4 beschrieben.

### Abbildung 5

Expressionsvektor pSXK 2 zur Expression von HIV-1 Antisense-RNA unter der Kontrolle des Cytomegalovirus Promotors (CMV), Das provirale HIV-1 DNA Fragment aus der mittleren genomischen Region des HIV umfaßt in diesem Beispiel nicht Sequenzen des 5'-Bereiches wie in den Beispielen 1 bis 4, sondern eine Sequenz aus der POL/VIF Region. Einzelheiten zum Aufbau des Vektors sind in Beispiel 5 aufgeführt.

### Abbildung 6

Übersichtsdiagramm zur Darstellung der HIV-1 Genfragmente, die in 3'→5' Antisense-Orientierung (Pfeilrichtung) in den Antisense-RNA Expressionsvektoren mit der in Spalte 1 angegebenen Bezeichnung enthalten sind, Der Promotor des jeweiligen Expressionsvektors ist in Spalte 2 angegeben, die Nukleotidnumerierung der HIV-Sequenz nach UWGCG GENEMBL DATA BANK File: HIVHXB2CG.VIRAL ist in Spalte 3 angegeben.

### Abbildung 7

Southern Blot Analyse von chromosomaler DNA aus einer klonierten U 937 Transfektante nach PCR-Amplifizierung, die den Expressionsvektor pSXK1 integriert in chromosomale DNA enthält. Aufgrund der Fragmentgröße der PCR-Produkte und der Hybridisierung mit einer HIV-Gensonde, die nicht die Primersequenz der PCR-Produkte enthält, wird die Unversehrtheit der Promotor/Insert-Sequenz anzeigt.
1 Kontrollansatz Vektor, 5'-Übergang, 887 Basenpaare Fragment
2 Kontrollansatz Vektor, 3'-Übergang, 775 Basenpaare Fragment
3 Chromosomale DNA, 5'-Übergang, 887 Basenpaare Fragment
4 Chromosomale DNA, 3'-Übergang. 775 Basenpaare Fragment
   Weitere Einzelheiten sind in Beispiel 9 genannt.

### Abbildung 8

Nachweis des HIV-1 Genfragments in chromosomaler DNA aus klonierten HUT 78 Transfektanten mittels PCR-Amplifikation. Die PCR-Reaktion wurde über ein Agarose-TAE-Gel aufgetrennt und nach Ethidiumbromid-Färbung unter UV-Licht photographiert. Das PCR-Produkt umfaßt 561 bp und entspricht dem HIV-1 Genfragment (Nukleotide 699-1305), welches auch Bestandteil des pSXK1 Vektors ist. Das 561 bp lange PCR-Produkt ist durch einen Pfeil gekennzeichnet.
- M =: DNA Längenstandard, 516 bp und 1018 bp sind gekennzeichnet
- 1 =: negativ Kontrolle, keine DNA wurde in die PCR-Reaktion gegeben
- 2 =: chromosomale DNA aus U937 Zellen (Wildtyp)
- 3 =: chromosomale DNA aus HUT 78 Zellen (Wildtyp)
- 4 =: negativ Kontrolle, keine DNA wurde in die PCR-Reaktion gegeben
- 5 =: positiv Kontrolle
- 6 =: chromosomale DNA aus HUT 78 Transformanten (SSHUT 6/1 107-9/1)
- 7 =: chromosomale DNA aus HUT 78 Transformanten (SSHUT 6/1 107-9/1)
- 8 =: chromosomale DNA aus HUT 78 Transformanten (SSHUT 6/1 107-9/1)
- 9 =: chromosomale DNA aus HUT 78 Transformanten (SSHUT 6/1 107-9/1)

### Abbildung 9

Nachweis des HIV-1 Genfragments (Nukleotide 4158-5792) in chromosomaler DNA aus U937 Monozyten- und HUT 78 T-Lymphozyten-Zellen mittels PCR-Amplifikation. Die Zellen wurden zuvor mit dem pSXK2-Vektor transfiziert. Die PCR-Reaktion wurde über ein Agarose-TAE-Gel aufgetrennt und nach Ethidiumbromid-Färbung unter UV-Licht photographiert. Das PCR-Produkt umfaßt das gesamte HIV-1 Genfragment in pSXK2 (Nukleotide 4158-5792) und ist 1811 bp lang. Das 1811 bp lange PCR-Produkt ist durch einen Pfeil gekennzeichnet.
- M =: DNA Längenstandard, 1635 bp und 2036 bp Längenstandards sind gekennzeichnet
- 1 =: negativ Kontrolle, keine DNA wurde in die PCR-Reaktion eingesetzt
- 2 =: chromosomale DNA aus U937 Zellen (Wildtyp)
- 3 =: chromosomale DNA aus HUT 78 Zellen (Wildtyp)
- 4 =: chromosomale DNA aus HUT 78 Transformanten (SSHUT 2/1 107-12/1)
- 5 =: chromosomale DNA aus U937 Transformanten (SSUK 3/1 107-12/1)

### Abbildung 10

Northern Blot Analyse zum Nachweis von mehreren Antisense-RNA Transkripten in einer transfizierten, klonierten U 937 Zellinie, die den Expressionsvektor KTX 11 in der Zellinie SSUK 20/15-KTX11b enthält. Mit einer Gensonde, die der HIV-1 Insert DNA des Vektors KTX 11 entspricht, lassen sich in der RNA der virusresistenten Zellinie (Spur 1) mindestens drei Antisense-RNA Transkripte mit 800 bp, 1 200 bp und 3 200 bp Länge nachweisen. Spur 2 enthält RNA aus der nicht transfizierten Zelline U 937 (Negativkontrolle). Spur 3 enthält RNA-Molekulargewichtsstandards.

### Abbildung 11

Verlauf der HIV-1 Antigenbildung über 60 Tage nach der Infektion (am Tag null) von HUT 78 Zellinien mit HIV-1 LAV/BRU Isolat. Auf der Ordinate ist die optische Dichte bei 450 nm (OD 450 nm) des HIV ELISA Testergebnisses vom Zellkulturüberstand angegeben. Bei einer optischen Dichte > 2,0 wurde der Kulturüberstand verdünnt und der angegebene OD-Wert aus der Verdünnung errechnet. Der cut-off Wert stellt die Nachweisgrenze der HIV Antigenbestimmung dar.

In den Antisense-RNA exprimierenden Zellinien SSHUT K16/2-SXK1 und SSHUT K1/1-SXK2 ist die HIV Replikation über einen Zeitraum von 60 Tagen vollständig gehemmt.

In der Zellinie HUT K14-KTX11 setzt die HIV Replikation verzögert gegenüber der nicht transfizierten Wildtyp-Zellinie HUT 78 ein.

### Abbildung 12

Verlauf der HIV-1 Antigenbildung über 60 Tage nach der Infektion (am Tag null) von U937 Zellinien mit HIV-1 LAV/BRU Isolat. Auf der Ordinate ist die optische Dichte bei 450 nm (OD 450 nm) des HIV ELISA Testergebnisses vom Zellkulturüberstand angegeben. Bei einer optischen Dichte > 2,0 OD-Einheiten wurde der Kulturüberstand verdünnt und der OD-Wert aus der Verdünnung errechnet. Der cut-off Wert stellt die Nachweisgrenze der HIV-Antigenbestimmung dar.

In der Antisense-RNA exprimierenden Zellinie SSUK3/1-SXK2 ist die HIV-Replikation über einen Zeitraum von 60 Tagen vollständig gehemmt, die anderen transfizierten Zellinien zeigen eine verzögerte HIV Replikation im Vergleich zur Wildtypzellinie (U937).

### Abbildung 13

PCR-Analytik zum Nachweis von HIV-1 DNA in Zellinien, die Antisense-RNA Expressionsvektoren enthalten und 60 Tage nach der HIV-Infektion analysiert wurden. Das Ethidiumbromid gefärbte Elektrophorese-Agarosegel zeigt die PCR-Produkte aus chromosomaler DNA der unten genannten Zellinien.

Mit dem Primerpaar 5'-ATG GAG CCA GTA GAT CCT-3' und 5'-TCT GT TCT ACC ATG TCAT wurde in HIV infizierten Zellinien ein 702 Basenpaare langes PCR-Fragment aus dem TAT-ENV Genbereich amplifiziert. Zum Vergleich mit dem PCR-Ergebnis ist in Klammern das Ergebnis des HIV-Antigentest der entsprechenden Zellkulturprobe aufgeführt.
- Spur M:: Molekulargewichtstandard, 600 bp ist markiert
- Spur 1:: HUT K14-KTX11 702 bp positiv (Antigentest positiv).
- Spur 2:: SSHUT K16/2-SXK1 702 bp positiv (Antigentest negativ).
- Spur 3:: PCR-Produkt aus Parallel-Infektionsversuch wie Spur 2
- Spur 4:: PCR-Produkt aus Parallel-Infektionsversuch wie Spur 2
- Spur 5:: SSHUT K1/1-SXK2 702 bp positiv (Antigentest negativ)
- Spur 6:: PCR-Produkt aus Parallel-Infektionsversuch wie Spur 5
- Spur 7:: Parallel-Infektionsversuch wie Spur 5
- Spur 8:: HUT 78, Positiv-Kontrolle
- Spuren 9-11:: SSUK3/1-SXK2 Kulturen: kein 702 bp PCR-Produkt nachweisbar (Antigentest negativ)

### Abbildung 14

Verlauf der HIV Antigenbildung über 30 Tage nach der Infektion (am Tag null) von HUT 78 Zellinien mit HIV-2 D194 Isolat. Auf der Ordinate ist die optische Dichte bei 450 nm (OD 450 nm) des HIV ELISA Testergebnisses vom Zellkulturüberstand angegeben. Bei einer optischen Dichte > 2,0 wurde der Kulturüberstand verdünnt und der angegebene OD-Wert aus der Verdünnung errechnet. Der cut-off Wert stellt die Nachweisgrenze der HIV-Antigenbestimmung dar.

In der Antisense-RNA exprimierenden Zellinie SSHUT K1/1-SXK2 ist die HIV-Replikation über einen Zeitraum von 14 Tagen vollständig gehemmt, die anderen transfizierten Zellinien zeigen eine verzögerte HIV Replikation im Vergleich zur Wildtypzellinie (HUT 78).

### Abbildung 15

Verlauf der HIV Antigenbildung über 30 Tage nach der Infektion (am Tag null) von U937 Zellinien mit HIV-2 D194 Isolat.

Auf der Ordinate ist die optische Dichte bei 450 nm (OD 450 nm) des HIV ELISA Testergebnisses vom Zellkulturüberstand angegeben. Bei einer optischen Dichte > 2,0 wurde der Kulturüberstand verdünnt und der angegebene OD-Wert aus der Verdünnung errechnet. Der cut-off Wert stellt die Nachweisgrenze der HIV-Antigenbestimmung dar.

In der Antisense-RNA exprimierenden Zellinie SSHUT K3/1-SXK2 ist die HIV-Replikation über einen Zeitraum von 30 Tagen vollständig gehemmt, die andere transfizierte Zellinie zeigt eine verzögerte HIV Replikation im Vergleich zur Wildtypzellinie (U937).

## Patentansprüche

1. Expressionsvektorkonstrukte zur Expression von Antisense-RNA, dadurch gekennzeichnet, daß sie eine Promotorgensequenz enthalten und eine HIV-1 provirale DNA in Antisense-Orientierung der Nukleotide 7514 → 474 oder 5786 → 474 oder 2096 → 474 oder 3825 → 681 oder 5786 → 4158 oder 5746 → 4648 oder 2369 → 1635 oder 3227 → 3003 oder 2099 → 678 oder 3829 → 2099 oder 4647 → 4157 oder 4157 → 3830 enthalten.

2. Expressionsvektorkonstrukte nach Anspruch 1, enthaltend provirale Gensequenzen von HIV-1 der Nukleotide 605 → 455 oder 670 → 440 oder 825 → 535 oder 6070 → 5800 oder 5640 → 5020 oder 5600 → 5040 oder 8690 → 8300 oder 9160 → 8800.

3. Expressionsvektorkonstrukte gemäß den Ansprüchen 1 und 2, dadurch gekennzeichnet, daß sie Zwei, drei oder mehr subgenomische Gensequenzen von einem Virus oder von mehreren Viren enthalten.

4. Transfizierte Zellen, die Expressionsvektorkonstrukte der Ansprüche 1 bis 3 enthalten.

5. Verwendung von Expressionsvektorkonstrukten gemäß den Ansprüchen 1 bis 3 zur Herstellung von Arzneimitteln.

6. Arzneimittel enthaltend einen oder mehrere Expressionsvektorkonstrukte aus den Ansprüchen 1 bis 3.
